# EUROPEAN PATENT APPLICATION

(11) **EP 4 119 117 A1**
(43) Date of publication of application: **18.01.2023**
(21) Application number: 21768668.2
(22) Date of filing: 09.03.2021
(51) Int. Cl.: A61H 15/02, A61H 39/06, A61F 7/00, A61H 39/04, A61H 15/00, A61H 1/00

(54) **HYPERTHERMO-THERAPEUTIC APPARATUS CAPABLE OF CONTROLLING MOVEMENT OF HEATING AND RUBBING DEVICE**

(30) Priority: 10.03.2020 KR 20200029752
(71) Applicant: Ceragem Co., Ltd., Cheonan-si, Chungcheongnam-do 31045 (KR)
(72) Inventor: LEE, Dong Myoung, Asan-si Chungcheongnam-do 31416 (KR); KIM, Ki Sung, Cheonan-si Chungcheongnam-do 31175 (KR); HAN, Sang Cheol, Cheonan-si Chungcheongnam-do 31179 (KR)
(74) Representative: Treeby, Philip David William
(86) International application number: PCT/KR2021/002923
(87) International publication number: WO 2021/182853

(57) **Abstract**

The present invention relates to a thermo-therapeutic apparatus capable of controlling movement of a heating moxibustion device. More specifically, the present invention relates to a thermo-therapeutic apparatus capable of controlling movement of a heating moxibustion device which can intensively massage a part desired by the user, can massage in various ways such as moxibustion or thermal massage, and can quickly move to a location desired by the user. To this end, a thermo-therapeutic apparatus capable of controlling movement of a heating moxibustion device includes a heating moxibustion device, a driving unit for moving the heating moxibustion device, a control unit for controlling an operation of the driving unit, and an input unit for selecting a thermal massage pattern desired by a user, wherein the control unit is equipped with an automatic mode in which when the user inputs a desired location, the heating moxibustion device is controlled to automatically move to the location to perform a thermal massage.

## Description

### TECHNICAL FIELD

The present invention relates to a thermo-therapeutic apparatus capable of controlling movement of a heating moxibustion device. More specifically, the present invention relates to a thermo-therapeutic apparatus capable of controlling movement of a heating moxibustion device which can intensively massage a part desired by the user, can massage in various ways such as moxibustion or thermal massage, and can quickly move to a location desired by the user.

### BACKGROUND

Conventionally, a thermotherapy bed has been widely used that moves along the body part and improves blood circulation through stimulation by the heat to painful area in order to alleviate acute or chronic pain that occurs in the muscles and nerve tissues of the spine region caused by continuing work in an inappropriate posture for a long time or becoming a habit of this posture for a long time, to improve blood circulation in the body or to relieve momentary muscle stiffness.

The conventional thermo-therapeutic apparatus used for such thermotherapy performs a massage while moving along the user's body in a longitudinal direction, and it is configured to be able to perform massage only with a massage pattern set at the time of manufacture while repeatedly reciprocating the entire movement section.

That is, although it is suitable for massaging the user's body as a whole, there is a limitation in that it is not possible to intensively massage only a specific part desired by the user.

Therefore, there is a need for improvement in this area.

The above information disclosed in this Background section is only for enhancement of understanding of the background of the invention and it may therefore contain information that does not form the prior art that is already known to a person of ordinary skill in the art.

### [Related Art Document]

### [Patent Document]

(Patent Document 1) Korean Utility Model Registration No. 20-0250827 (registered 2001.10.05)

### SUMMARY

The present invention is directed to providing a thermo-therapeutic apparatus capable of controlling movement of a heating moxibustion device which can intensively massage a part desired by the user, can massage in various ways such as moxibustion or thermal massage, and can quickly move to a location desired by the user.

The technical problems to be solved in the present invention are not limited thereto, and other technical problems not mentioned will be clearly understood by those of ordinary skill in the art from the following description.

A thermo-therapeutic apparatus capable of controlling movement of a heating moxibustion device according to the present invention includes a heating moxibustion device, a driving unit for moving the heating moxibustion device, a control unit for controlling an operation of the driving unit, and an input unit for selecting a thermal massage pattern desired by a user, wherein the control unit is equipped with an automatic mode in which when the user inputs a desired location, the heating moxibustion device is controlled to automatically move to the location to perform a thermal massage.

In this case, the control unit is further equipped with a manual mode in which when the heating moxibustion device moves along the user's longitudinal direction to a location desired by the user the user directly controls the driving unit to perform a thermal massage.

In this case, the manual mode may include a manual 1 mode in which the user changes a height of the heating moxibustion device to a height desired by the user in a location desired by the user and then an operation of applying moxibustion is performed in a way of fixing the vertical height of the heating moxibustion device, and a manual 2 mode configured to perform one or more of an operation in which after fixing the vertical height of the heating moxibustion device to a user's desired height at a location desired by the user, massage operation is performed by repeatedly moving the heating moxibustion device a predetermined distance in the longitudinal direction or an operation in which after fixing the heating moxibustion device in the longitudinal direction, acupressure operation is performed by repeatedly moving the heating moxibustion device a predetermined distance in the height direction only to the height desired by the user, or configured to perform one or more of the massage operation or the acupressure operation along with the operation of applying the moxibustion.

In this case, the automatic mode may include an automatic 1 mode in which after automatically moving the heating moxibustion device to a location desired by the user and changing a height of the heating moxibustion device to a height desired by the user in a location desired by the user and then an operation of applying moxibustion is performed in a way of fixing the vertical height of the heating moxibustion device, and an automatic 2 mode configured to perform one or more of an operation in which after automatically moving the heating moxibustion device to a location desired by the user and fixing the vertical height of the heating moxibustion device to a user's desired height at the location desired by the user, massage operation is performed by repeatedly moving the heating moxibustion device a predetermined distance in the longitudinal direction or an operation in which after fixing the heating moxibustion device in the longitudinal direction, acupressure operation is performed by repeatedly moving the heating moxibustion device a predetermined distance in the height direction only to the height desired by the user, or configured to perform one or more of the massage operation or the acupressure operation along with the operation of applying the moxibustion.

In this case, when the user selects the automatic mode and inputs a desired location, the control unit may scan a shape of the user's spine while moving the heating moxibustion device in the user's longitudinal direction and then move the heating moxibustion device to the location desired by the user.

In this case, when a first scan of scanning the shape of the spine while moving from one side of the user's longitudinal direction to the other side is performed and then a second scan of scanning the shape of the spine while moving from the other side of the user's longitudinal direction to one side is performed, after the first scan and the second scan are finished the control unit may control to perform a thermal massage by moving the heating moxibustion device to a location desired by the user.

In this case, when the first scan of scanning the shape of the spine while moving from one side of the user's longitudinal direction to the other side is finished, the control unit may control the heating moxibustion device to perform a thermal massage by moving it to a location desired by the user.

In this case, when the first scan of scanning the shape of the spine while moving from one side of the user's longitudinal direction to the other side is finished, the control unit may control the heating moxibustion device to perform a thermal massage by moving it to a location desired by the user after moving the heating moxibustion device to one side of the user's longitudinal direction.

In this case, the control unit may control so that a movement speed at which the heating moxibustion device moves to a location desired by the user and an operation speed at which the heating moxibustion device performs a thermal massage at the location desired by the user are different, according to the manual mode and the automatic mode.

In this case, the control unit may control the heating moxibustion device to move in a state where the vertical height of the heating moxibustion device is adjusted so that the user can recognize the moving location of the heating moxibustion device when moving the heating moxibustion device according to the manual mode.

In this case, the control unit may control the heating moxibustion device to move in a state where the vertical height of the heating moxibustion device is lowered when moving the heating moxibustion device to a location desired by the user.

In this case, the control unit may control to reconfirm whether the moved location of the heating moxibustion device is the location desired by the user when moving the heating moxibustion device to the location desired by the user according to the automatic mode.

In this case, the control unit may control to compare a reference acupressure pressure at the location identified in the scanning process with a new acupressure pressure at a location where the heating moxibustion device has moved after the scan, and if the reference acupressure pressure and the new acupressure pressure are the same, to perform a thermal massage according to the automatic mode, and if the reference acupressure pressure and the new acupressure pressure are different, after confirming the location where the new acupressure pressure matches the reference acupressure pressure while repeatedly moving a certain distance along the longitudinal direction based on the location where the heating moxibustion device has moved, to perform a thermal massage according to the automatic mode.

The thermo-therapeutic apparatus capable of controlling movement of a heating moxibustion device according to the present invention having such configurations performs intensively massage after the user moves the heating moxibustion device to a location desired by the user by using the manual mode while directly feeling the moving location of the heating moxibustion device, thereby maximizing the massage effect.

In addition, the massage effect may be maximized and user convenience may be improved by intensively performing the massage after automatically moving the heating moxibustion device to a location desired by the user by using the automatic mode.

In particular, since an effect through moxibustion or a thermal massage effect can be obtained according to each mode, a massage effect may be maximized.

In addition, in the automatic mode, since after scanning the shape of the user's spine, the heating moxibustion device is automatically moved to a location desired by the user, it may be possible to move it to an accurate location.

Advantageous effects of the present invention are not limited to the above-described effects, and should be understood to include all effects that can be inferred from the configuration of the invention described in the detailed description or claims of the present invention.

### BRIEF DESCRIPTION OF THE DRAWINGS

The above and other aspects, features, and advantages of the present invention will become more apparent to those of ordinary skill in the art by describing embodiments thereof in detail with reference to the accompanying drawings, in which:
FIG. 1 is a cross-sectional view showing a thermo-therapeutic apparatus capable of controlling movement of a heating moxibustion device according to the present invention;
FIG. 2 is a schematic diagram showing a configuration of a control unit according to the present invention;
FIG. 3 to 5 are flow charts showing various embodiments in which the thermo-therapeutic apparatus is operated according to an automatic mode applied to the present invention;
FIG. 6 is a cross-sectional view showing a state in which the thermo-therapeutic apparatus is operated according to an automatic mode applied to the present invention.

### Description of Symbols

| | | | |
|---|---|---|---|
| 100: | heating moxibustion device | 101: | main mat |
| 102: | auxiliary mat | 103: | mounting unit |
| 200: | driving unit | 210: | driving motor |
| 211: | conveying member | 300: | control unit |
| 310: | manual mode | 311: | manual 1 mode |
| 312: | manual 2 mode | 320: | automatic mode |
| 321: | automatic 1 mode | 322: | automatic 2 mode |
| 400: | input unit | x: | longitudinal direction |
| y: | height direction | v1: | movement speed |
| v2: | operation speed | h: | vertical height |

### DETAILED DESCRIPTION OF THE EMBODIMENTS

Hereinafter, exemplary embodiments of the present invention will be described in detail so that those of ordinary skill in the art can readily implement the present invention with reference to the accompanying drawings. The present invention may be embodied in many different forms and are not limited to the embodiments set forth herein. In the drawings, parts unrelated to the description are omitted for clarity. Throughout the specification, like reference numerals denote like elements.

It is understood that the terms "comprise" or "have" when used in this specification, are intended to specify the presence of stated features, integers, steps, operations, members, components and/or a combination thereof but not preclude the possibility of the presence or addition of one or more other features, integers, steps, operations, members, components, or a combination thereof. In addition, it will be understood that when an element such as a layer, film, region, or substrate is referred to as being "on" another element, it can be "directly on" the other element or intervening elements may also be present. Conversely, it will be understood that when an element such as a layer, film, region, or substrate is referred to as being "below" another element, it can be "directly below" the other element or intervening elements may also be present.

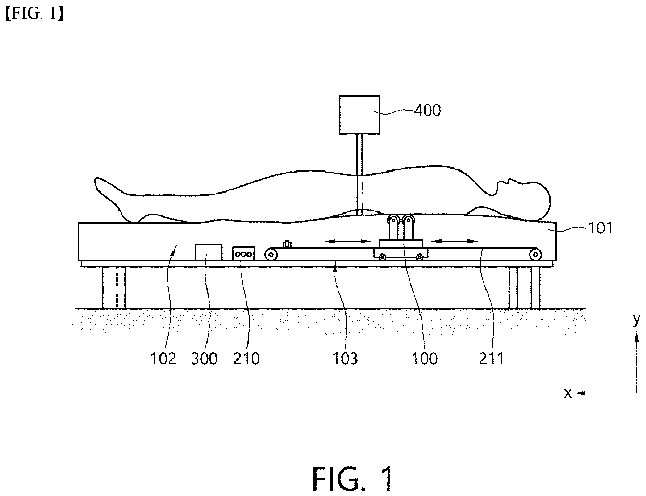

FIG. 1 is a cross-sectional view showing a thermo-therapeutic apparatus capable of controlling movement of a heating moxibustion device according to the present invention.

As shown in FIG. 1, a thermo-therapeutic apparatus capable of controlling movement of a heating moxibustion device according to the present invention include a heating moxibustion device 100, a driving unit 200 for moving the heating moxibustion device 100, a control unit 300 for controlling the operation of the driving unit 200, and an input unit 400 for selecting a thermal massage pattern desired by the user.

The thermo-therapeutic apparatus capable of controlling movement of a heating moxibustion device may include a main mat 101 used for the upper body and spine of the user and an auxiliary mat 102 used for the lower body of the user. In addition, it may include a mounting unit 103 used for mounting and supporting the main mat 101 and the auxiliary mat 102, as necessary.

The heating moxibustion device 100 may massage a user's spine while moving along the spine in the longitudinal direction (x), and the heating moxibustion device 100 may provide a user with a thermal poultice effect and a massage effect by using high temperature heat and far infrared radiation, etc. generated by using electrical energy supplied from a power supply unit (not shown). The heating moxibustion device 100 may be either a cap type or roller type depending on the shape, but is not limited thereto, and various shapes and structures are possible. Such heating moxibustion device 100 includes a heating unit that generates heat using electric energy and a heat transmission unit heated by the heating unit. Here, the heating unit may include a lamp or electric heater and the heat transmission unit may include a far-infrared radiating material, but they are not limited thereto, and various heating elements or materials and substances that can be heated by heat may be used.

In addition, the driving unit 200 may include a first driving member for moving the heating moxibustion device 100 in the user's longitudinal direction (x). The first driving member may include a driving motor 210 and a conveying member 211 for reciprocating the heating moxibustion device 100. The driving motor 210 rotates by receiving electric energy, and the conveying member 211 is connected to the driving motor to transfer rotational force according to the rotation of the driving motor 210 to move the heating moxibustion device 100. The conveying member 211 is connected to the heating moxibustion device 100 and is used to convey the heating moxibustion device 100 in one or the other direction along the user's longitudinal direction (x) according to the forward or reverse rotation of the driving motor 210. The conveying member 211 may use optionally any of a conveying belt, conveying chain, and conveying rope, but is not limited thereto, and various means for conveying subjects using the driving force of the driving motor 210 such as a method using a rack and a pinion may be used. The driving motor 210 may be configured to provide a driving force in a state spaced apart from the heating moxibustion device 100 or to provide a driving force in a state inserted into the heating moxibustion device 100.

In addition, the driving unit 200 may include a second driving member that raises the vertical height of the heating moxibustion device 100 to apply a pressing force to the user, or lowers the vertical height of the heating moxibustion device 100 so that the pressing force is removed.

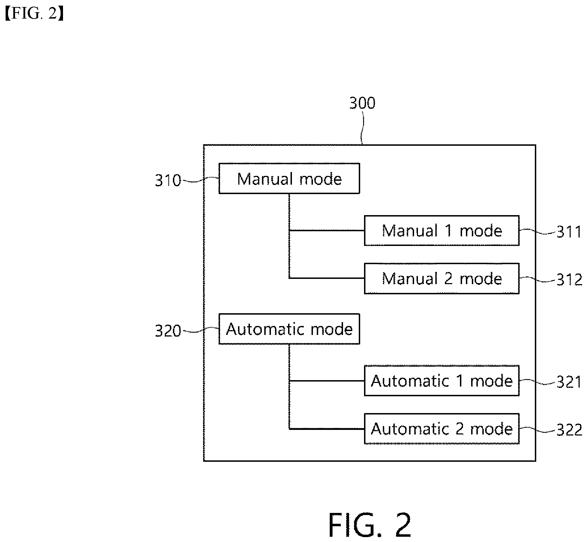

FIG. 2 is a schematic diagram showing a configuration of a control unit according to the present invention.

As shown in FIG. 2, the control unit 300 receives the following inputs: a manual mode 310 in which when the heating moxibustion device 100 moves along the user's longitudinal direction (x) to a location desired by the user, the user directly controls the driving unit 200 to perform a thermal massage, and an automatic mode 320 in which when the user inputs a desired location, the heating moxibustion device 100 is controlled to automatically move to the location to perform a thermal massage.

That is, the user can select the manual mode 310 using the input unit 400; after the manual mode 310 is selected, the user moves the heating moxibustion device 100 while directly feeling the moving location of the heating moxibustion device 100; and when the heating moxibustion device 100 is moved to a location desired by the user, the heating moxibustion device performs a thermal massage intensively in the location, thereby maximizing the massage effect.

In addition, the user can select the automatic mode 320 using the input unit 400; and when the user selects the automatic mode 320 and then inputs a desired location, the controller 300 automatically moves the heating moxibustion device 100 to the location desired by the user, and then controls the heating moxibustion device to intensively perform a thermal massage.

The general human spine is largely classified into cervical vertebra, thoracic vertebra, lumbar vertebra, and sacrum/coccyx. The cervical vertebrae represent the 1st to 7th bones and are related to autonomic nerve functions such as headache, insomnia, optic nerve, earache, tinnitus, and dizziness. The thoracic vertebrae represent the 8th to 19th bones and are closely related to autonomic nerve functions such as heart function, digestive function, vasoconstriction, and internal diseases. The lumbar vertebrae represent 20th to 24th bones and are related to autonomic nerve functions such as colitis, diarrhea, constipation, lower abdominal pain, low back pain, sciatica, prostate and bladder disease. The sacrum/coccyx vertebrae represent 25th to 30th bones and are related to autonomic nerve functions such as genitals, prostate, hemorrhoids, and rectum. Such relationship between the position of the spine and the body is previously input to the control unit 300, and with this the user can specify the desired position for massage and move the heating moxibustion device 100 to the specific spine position to perform a thermal massage, thereby not only maximizing the massage effect but also improving user convenience.

The above-described manual mode 310 includes a manual 1 mode 311 in which the user changes a height of the heating moxibustion device 100 along the height direction (y) to a height desired by the user in a location desired by the user and then an operation of applying moxibustion is performed in a way of fixing the vertical height of the heating moxibustion device 100. That is, as the height of the heating moxibustion device 100 increases, the intensity of the thermal massage increases, so the height desired by the user means the height of the heating moxibustion device 100 capable of performing a thermal massage at the intensity selected by the user.

In addition, the manual mode 310 may include a manual 2 mode 312 that controls the heating moxibustion device 100 in a different way from the manual 1 mode 311 described above. That is, the manual 2 mode 312 may be configured to perform one or more of an operation in which after fixing the vertical height of the heating moxibustion device 100 to a user's desired height at a location desired by the user, massage operation is performed by repeatedly moving the heating moxibustion device a predetermined distance in the longitudinal direction (x), or an operation in which after fixing the heating moxibustion device 100 in the longitudinal direction (x), acupressure operation is performed by repeatedly moving the heating moxibustion device 100 a predetermined distance in the height direction (y) only to the height desired by the user. In other words, it is configured to perform each or both of a massage operation or an acupressure operation except for an operation of applying the moxibustion. Alternatively, it is possible to be configured to perform one or more of the massage operation or the acupressure operation along with the operation of applying the moxibustion. That is, it is also possible to be configured to perform the moxibustion operation and the massage operation, perform the moxibustion operation and the acupressure operation, or perform all of the moxibustion operation, the massage operation and the acupressure operation. In this case, it is preferable to operate the moxibustion operation, massage operation, and acupressure operation so that each operation can be distinguished.

That is, as described above, the user may select the manual mode 310 using the input unit 400, and after selecting the manual mode 310, the user may move the heating moxibustion device 100 while directly feeling the moving location of the heating moxibustion device 100. When the heating moxibustion device 100 is moved to a location desired by the user, the heating moxibustion device performs a thermal massage intensively in the location and the user can select the manual 1 mode 311 or the manual 2 mode 312. In the case of the manual 1 mode 311, it is a mode in which a moxibustion operation is performed at a location desired by the user; and in the case of the manual 2 mode 312, it is a mode in which a massage operation or acupressure operation, or both a massage operation and acupressure operation are performed, a moxibustion and a massage operation are performed, a moxibustion operation and an acupressure operation are performed, or all of a moxibustion operation, a massage operation, and an acupressure operation are performed at a location desired by the user, a single operation or a plurality of operations may be included, and the user can select a mode to get an appropriate thermal massage at a desired location.

The above-described automatic mode 320 includes an automatic 1 mode 321 in which after automatically moving the heating moxibustion device 100 to a location desired by the user and changing a height of the heating moxibustion device 100 to a height desired by the user in a location desired by the user and then an operation of applying moxibustion is performed in a way of fixing the vertical height of the heating moxibustion device 100.

In addition, the automatic mode 320 may include an automatic 2 mode 322 that controls the heating moxibustion device 100 in a different way from the automatic 1 mode 321 described above. That is, the automatic 2 mode 322 may be configured to perform one or more of an operation in which after automatically moving the heating moxibustion device 100 to a location desired by the user and fixing the vertical height of the heating moxibustion device 100 to a user's desired height at the location desired by the user, massage operation is performed by repeatedly moving the heating moxibustion device a predetermined distance in the longitudinal direction (x), or an operation in which after fixing the heating moxibustion device 100 in the longitudinal direction (x), acupressure operation is performed by repeatedly moving the heating moxibustion device 100 a predetermined distance in the height direction (y) only to the height desired by the user. In other words, it is configured to perform each or both of a massage operation or an acupressure operation except for an operation of applying the moxibustion. Alternatively, it is possible to be configured to perform one or more of the massage operation or the acupressure operation along with the operation of applying the moxibustion. That is, it is also possible to be configured to perform the moxibustion operation and the massage operation, perform the moxibustion operation and the acupressure operation, or perform all of the moxibustion operation, the massage operation and the acupressure operation. In this case, it is preferable to operate the moxibustion operation, massage operation, and acupressure operation so that each operation can be distinguished.

That is, as described above, the user may select the automatic mode 320 using the input unit 400, and after selecting the automatic mode 320, the user may select a desired location for massage. When the heating moxibustion device 100 is automatically moved to a location desired by the user, the heating moxibustion device performs a thermal massage intensively in the location and the user can select the automatic 1 mode 321 or the automatic 2 mode 322. In the case of the automatic 1 mode 321, it is a mode in which after automatically moving the heating moxibustion device 100 to a location desired by the user a moxibustion operation is performed at the location desired by the user; and in the case of the automatic 2 mode 322, it is a mode in which after automatically moving the heating moxibustion device 100 to a location desired by the user a massage operation or acupressure operation, or both a massage operation and acupressure operation are performed, a moxibustion and a massage operation are performed, a moxibustion operation and an acupressure operation are performed, or all of a moxibustion operation, a massage operation, and an acupressure operation are performed at a location desired by the user, a single operation or a plurality of operations may be included, and the user can select a mode to get an appropriate thermal massage at a desired location.

In this way, since an effect through moxibustion or a thermal massage effect can be obtained according to each mode, a massage effect may be maximized.

As described above, when a user selects an automatic mode 320 and inputs a desired location, the control unit 300 scans a shape of the user's spine while moving the heating moxibustion device 100 in the user's longitudinal direction (x), and then moves the heating moxibustion device 100 to a location desired by the user and performs a thermal massage.

That is, the control unit 300 first scans the shape of the user's spine before moving the heating moxibustion device 100 to a location desired by the user, and moves the heating moxibustion device 100 to a location desired by the user based on such scan information.

In this case, it may be configured that the user's spine shape is generated as data not only for the spine shape for the entire spine section including the above-described cervical vertebra, thoracic vertebra, lumbar vertebra, and sacrum/coccyx, but also for the spine shape for some sections and then saved in the control unit 300.

That is, in the automatic mode 320, since after scanning the shape of the user's spine, the heating moxibustion device 100 is automatically moved to a location desired by the user, it may be possible to perform a thermal massage by moving it to an accurate location.

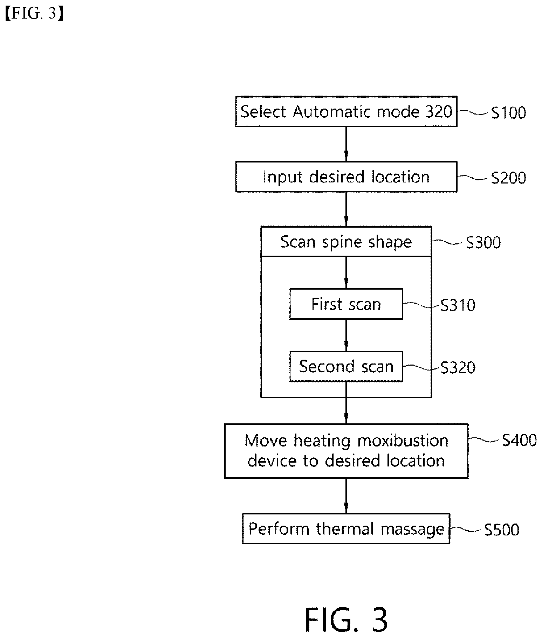

FIG. 3 to 5 are flow charts showing various embodiments in which the thermo-therapeutic apparatus is operated according to an automatic mode applied to the present invention. That is, when the user selects the automatic mode 320 at step S100 and inputs a desired location at step S200, the spine shape is scanned at step S300, which will be described in detail as follows.

First, as shown in FIG. 3, when a first scan of scanning the shape of the spine while moving from one side of the user's longitudinal direction (x) to the other side is performed at step S310 and then a second scan of scanning the shape of the spine while moving from the other side of the user's longitudinal direction (x) to one side is performed at step S320, after the first scan at step S310 and the second scan at step S320 are finished, the control unit 300 may control the heating moxibustion device 100 to perform a thermal massage at step S500 by moving it to a location desired by the user at step S400.

That is, the first scan is performed from one side of the user to the other side at step S310, and then the second scan is performed from the other side of the user to one side at step S320, and one side of the user at which the second scan at step S320 is finished becomes a reference point and the heating moxibustion device 100 is moved automatically based thereon. In this way, if the user's spine shape is scanned using the first scan at step S310 and the second scan at step S320, it may be possible to accurately move to a desired location by the user by obtaining accurate spine shape data.

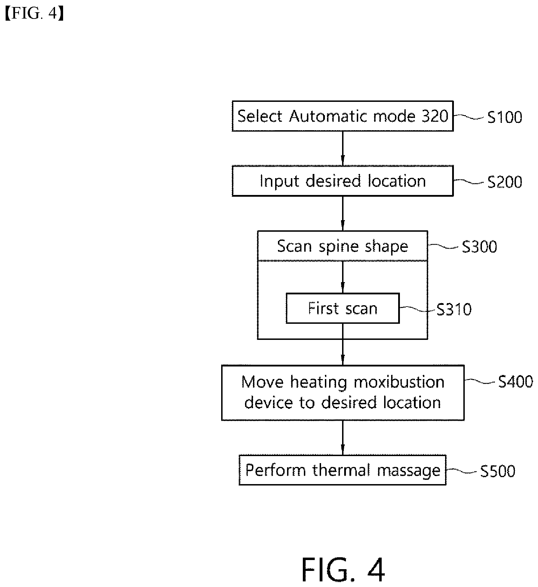

Alternatively, as shown in FIG. 4, when the first scan of scanning the shape of the spine while moving from one side of the user's longitudinal direction (x) to the other side is finished at step S310, the control unit 300 may control the heating moxibustion device 100 to perform a thermal massage at step S500 by moving it to a location desired by the user at step S400.

That is, only the first scan is performed from one side of the user to the other side at step S310, and the other side of the user at which the first scan at step S310 is finished becomes the reference point and the heating moxibustion device 100 is moved automatically based on thereon. In this way, if the heating moxibustion device 100 is moved directly from the location where the first scan at step S310 is finished to a location desired by the user, the waiting time for the user may be shortened, thereby improving the user's satisfaction.

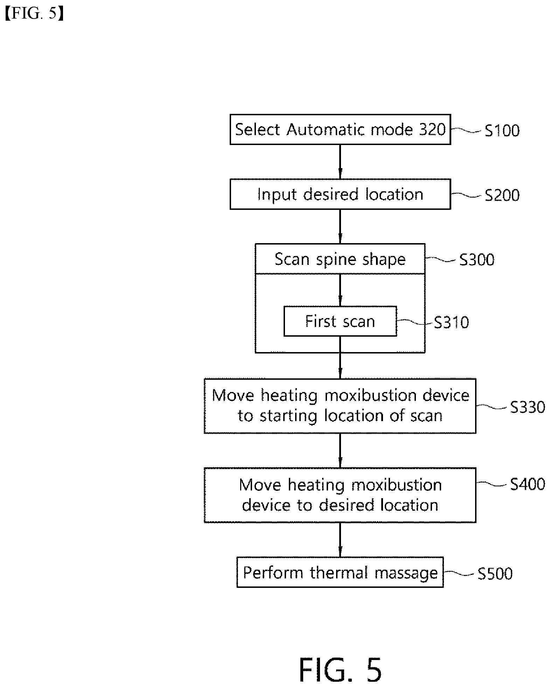

Alternatively, as shown in FIG. 5, when the first scan of scanning the shape of the spine while moving from one side of the user's longitudinal direction to the other side is finished at step S310, the control unit 300 may control the heating moxibustion device 100 to perform a thermal massage at step S500 by moving it to a location desired by the user at step S400 after moving the heating moxibustion device to one side of the user's longitudinal direction at step S330.

That is, only the first scan is performed from one side of the user to the other side at step S310, but when the first scan at step S310 is finished, the heating moxibustion device 100 is moved back to one side of the user to reset the location of the heating moxibustion device 100 and set one side of the user as a reference point, and the heating moxibustion device 100 is moved based on thereon. When configured in this way, it may be possible to move the heating moxibustion device 100 to an exact location desired by the user. However, it may take some time in the process of moving the heating moxibustion device 100 from the other side to one side of the user after the first scan at step S310, but as will be described later, if the heating moxibustion device 100 is moved at high speed after the vertical height of the heating moxibustion device 100 is lowered to the bottom dead center as much as possible, the time for the heating moxibustion device 100 to move from the other side of the user to one side after the first scan at step S310 may be minimized.

Meanwhile, the control unit 300 may control so that a movement speed v1 at which the heating moxibustion device 100 moves to a location desired by the user and an operation speed v2 at which the heating moxibustion device 100 performs a thermal massage at the location desired by the user are different, according to the manual mode 310 and the automatic mode 320. That is, by setting the movement speed v1 and the operation speed v2 of the heating moxibustion device 100 differently, the moving process of the heating moxibustion device 100 may be optimized and the thermal massage effect may be maximized.

In this case, in the case of the manual mode 310, it is preferable that the movement speed v1 of the heating moxibustion device 100 is set greater than the operation speed v2 of the heating moxibustion device 100. Through this, it may be possible to shorten the time required in the process of moving the heating moxibustion device 100 and maximize the thermal massage effect.

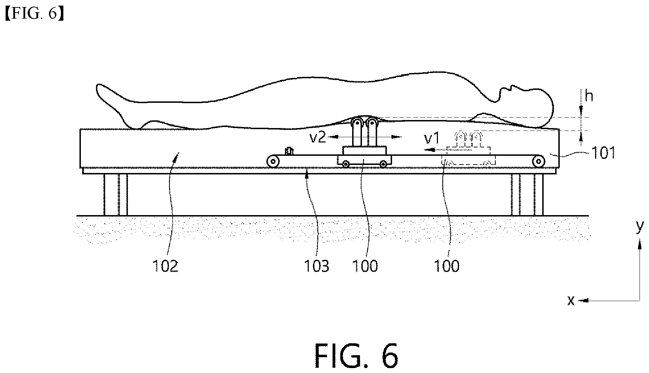

FIG. 6 is a cross-sectional view showing a state in which the thermo-therapeutic apparatus is operated according to an automatic mode applied to the present invention, as shown in FIG. 6, the control unit 300 may control so that the movement speed v1 at which the heating moxibustion device 100 moves to a location desired by the user and the operation speed v2 at which the heating moxibustion device 100 performs a thermal massage at the location desired by the user are different according to the automatic mode 320.

That is, as described above, according to the automatic mode 320, when moving the heating moxibustion device 100 to a location desired by the user, it is to shorten the user's waiting time by increasing the movement speed v1 to allow high speed movement, and in the case of performing the thermal massage at a location desired by the user, it is to maximize the massage effect by reducing the operation speed v2 of the heating moxibustion device 100.

In addition, as described above, when moving the heating moxibustion device 100 to a location desired by the user, it is preferable that the control unit 300 controls the heating moxibustion device 100 to move in a state where the vertical height h of the heating moxibustion device 100 is lowered to the bottom dead center as much as possible in order to prevent the user's body from colliding with the heating moxibustion device 100 moving at high speed.

On the other hand, in the case of the manual mode 310, since it is necessary to control the movement of the heating moxibustion device 100 in a state where the user directly recognizes the location of the heating moxibustion device 100, the control unit 300 controls to move in a state where the vertical height of the heating moxibustion device 100 is adjusted.

In this case, the control unit 300 may control the heating moxibustion device 100 to move in a state where the vertical height of the heating moxibustion device 100 is raised to the height of the heating moxibustion device 100 (a height desired by the user) capable of performing a thermal massage at an intensity selected by the user. When configured in this way, thermal massage may be possible even in the process of moving the heating moxibustion device 100.

Alternatively, the control unit 300 may also control the heating moxibustion device 100 to move in a state lower than the height desired by the user but raised to a height at which the user can recognize the moving location of the heating moxibustion device 100. When configured in this way, the user may accurately recognize the location of the heating moxibustion device 100.

Alternatively, if the user can recognize even in a state in which the heating moxibustion device is lowered to the lowest position (bottom dead center) due to the product characteristics of the thermo-therapeutic apparatus, the control unit 300 may also control the heating moxibustion device 100 to move in a state in which the heating moxibustion device 100 is descended to the lowest position. When configured in this way, the resistance caused by the user's body when the heating moxibustion device 100 moves is reduced, thereby minimizing the amount of electricity power consumption. However, since the height at which the heating moxibustion device 100 can be recognized varies according to the user's body shape, the control unit 300 may propose to allow the user to select a movement method suitable for his/her body shape by using the first scan S310 or the first scan S310 and the second scan S320 described above.

As described above, in the case of the automatic mode 320, when moving the heating moxibustion device 100 to a desired location after scanning, it is important to move it at high speed so as to shorten the waiting time of the user, to this end, it can be moved in a state where the vertical height of the heating moxibustion device 100 is lowered to the bottom dead center as far as possible. Which is to prevent the heating moxibustion device 100 moving at high speed from colliding with the user's body since in the case of the automatic mode 320, the heating moxibustion device 100 automatically moves so that the user does not need to recognize the location of the heating moxibustion device 100.

Alternatively, the control unit 300 may control the heating moxibustion device 100 to move in a state where the vertical height of the heating moxibustion device 100 is raised to the height of the heating moxibustion device 100 (a height desired by the user) capable of performing a thermal massage at an intensity selected by the user. That is, it is because it takes a certain amount of time in the process of lowering and raising the vertical height of the heating moxibustion device 100 for the movement of the heating moxibustion device 100 and if the moving distance of the heating moxibustion device 100 is long, it may be effective to move the heating moxibustion device 100 in a state where it is lowered to the bottom dead center due to reduction of electricity consumption for the driving motor 210, but if the moving distance of the heating moxibustion device 100 is short, it may be effective to move the heating moxibustion device 100 in a state where the vertical height of the heating moxibustion device 100 is fixed to a height desired by the user.

It is preferable that when moving the heating moxibustion device 100 the control unit 300 determines whether to move the heating moxibustion device 100 in a state where the vertical height of the heating moxibustion device 100 is fixed or in a state where the vertical height of the heating moxibustion device 100 is lowered to the bottom dead center by comparing the required time according to moving distance of the heating moxibustion device 100, the time required for descending and rising of the heating moxibustion device 100, and the electricity power consumption when moving the heating moxibustion device 100.

In addition, when configured in this way, thermal massage may be possible even in the process of moving the heating moxibustion device 100.

In addition, when moving the heating moxibustion device 100 to a location desired by the user according to the automatic mode 320, the control unit 300 may control to reconfirm whether the moved location of the heating moxibustion device 100 is the location desired by the user.

That is, this is because the position of each spine may be changed due to the user's movement in the process of moving the heating moxibustion device 100 after scanning the shape of the user's spine. Therefore, it is preferable to perform a thermal massage after confirming whether or not there has been a movement of the user.

As described above, it is important to check the movement of the user and move the heating moxibustion device 100. To this end, the control unit 300 controls to compare a reference acupressure pressure at the location identified in the scanning process with a new acupressure pressure at the location where the heating moxibustion device 100 has moved after the scan, and if the reference acupressure pressure and the new acupressure pressure are the same, to determine that there is no movement of the user and to perform a thermal massage according to the automatic mode 320, or if the reference acupressure pressure and the new acupressure pressure are different, the user's movement is present, so after confirming the location where the new acupressure pressure matches the reference acupressure pressure while repeatedly moving a certain distance along the longitudinal direction (x) based on the location where the heating moxibustion device 100 has moved, the control unit 300 controls to perform a thermal massage according to the automatic mode 320, whereby it is possible to perform a thermal massage at the correct location even when there is a user's movement.

Although exemplary embodiments of the present invention have been described, the spirit of the present invention is not limited to the embodiments set forth herein. Those of ordinary skill in the art who understand the spirit of the present invention may easily propose other embodiments through supplement, change, removal, addition, etc. of elements within the same spirit, but the embodiments will be also within the scope of the present invention.

## Claims

1. A thermo-therapeutic apparatus capable of controlling movement of a heating moxibustion device, comprising: a heating moxibustion device, a driving unit for moving the heating moxibustion device, a control unit for controlling an operation of the driving unit, and an input unit for selecting a thermal massage pattern desired by a user,
wherein the control unit is equipped with an automatic mode in which when the user inputs a desired location, the heating moxibustion device is controlled to automatically move to the location to perform a thermal massage.

2. The apparatus of claim 1, wherein the control unit is further equipped with a manual mode in which when the heating moxibustion device moves along the user's longitudinal direction to a location desired by the user, the user directly controls the driving unit to perform a thermal massage.

3. The apparatus of claim 2, wherein the manual mode comprises:
a manual 1 mode in which the user changes a height of the heating moxibustion device to a height desired by the user in a location desired by the user and then an operation of applying moxibustion is performed in a way of fixing the vertical height of the heating moxibustion device, and
a manual 2 mode configured to perform one or more of an operation in which after fixing the vertical height of the heating moxibustion device to a user's desired height at a location desired by the user, massage operation is performed by repeatedly moving the heating moxibustion device a predetermined distance in the longitudinal direction or an operation in which after fixing the heating moxibustion device in the longitudinal direction, acupressure operation is performed by repeatedly moving the heating moxibustion device a predetermined distance in the height direction only to the height desired by the user, or configured to perform one or more of the massage operation or the acupressure operation along with the operation of applying the moxibustion.

4. The apparatus of claim 1, wherein the automatic mode comprises:
an automatic 1 mode in which after automatically moving the heating moxibustion device to a location desired by the user and changing a height of the heating moxibustion device to a height desired by the user in a location desired by the user and then an operation of applying moxibustion is performed in a way of fixing the vertical height of the heating moxibustion device, and
an automatic 2 mode configured to perform one or more of an operation in which after automatically moving the heating moxibustion device to a location desired by the user and fixing the vertical height of the heating moxibustion device to a user's desired height at the location desired by the user, massage operation is performed by repeatedly moving the heating moxibustion device a predetermined distance in the longitudinal direction or an operation in which after fixing the heating moxibustion device in the longitudinal direction, acupressure operation is performed by repeatedly moving the heating moxibustion device a predetermined distance in the height direction only to the height desired by the user, or configured to perform one or more of the massage operation or the acupressure operation along with the operation of applying the moxibustion.

5. The apparatus of claim 1, wherein when the user selects the automatic mode and inputs a desired location, the control unit controls to scan a shape of the user's spine while moving the heating moxibustion device in the user's longitudinal direction and then move the heating moxibustion device to the location desired by the user and perform a thermal massage.

6. The apparatus of claim 5, wherein when a first scan of scanning the shape of the spine while moving from one side of the user's longitudinal direction to the other side is performed and then a second scan of scanning the shape of the spine while moving from the other side of the user's longitudinal direction to one side is performed, after the first scan and the second scan are finished the control unit controls to perform a thermal massage by moving the heating moxibustion device to a location desired by the user.

7. The apparatus of claim 5, wherein when the first scan of scanning the shape of the spine while moving from one side of the user's longitudinal direction to the other side is finished, the control unit controls the heating moxibustion device to perform a thermal massage by moving it to a location desired by the user.

8. The apparatus of claim 5, wherein when the first scan of scanning the shape of the spine while moving from one side of the user's longitudinal direction to the other side is finished, the control unit controls the heating moxibustion device to perform a thermal massage by moving it to a location desired by the user after moving the heating moxibustion device to one side of the user's longitudinal direction.

9. The apparatus of claim 2, wherein the control unit controls so that a movement speed at which the heating moxibustion device moves to a location desired by the user and an operation speed at which the heating moxibustion device performs a thermal massage at the location desired by the user are different, according to the manual mode and the automatic mode.

10. The apparatus of claim 2, wherein the control unit controls the heating moxibustion device to move in a state where the vertical height of the heating moxibustion device is adjusted so that the user can recognize the moving location of the heating moxibustion device when moving the heating moxibustion device according to the manual mode.

11. The apparatus of claim 1, wherein the control unit controls the heating moxibustion device to move in a state where the vertical height of the heating moxibustion device is lowered when moving the heating moxibustion device to a location desired by the user according to the automatic mode.

12. The apparatus of claim 5, wherein the control unit controls to reconfirm whether the moved location of the heating moxibustion device is the location desired by the user when moving the heating moxibustion device to the location desired by the user according to the automatic mode.

13. The apparatus of claim 12, wherein the control unit controls to compare a reference acupressure pressure at the location identified in the scanning process with a new acupressure pressure at a location where the heating moxibustion device has moved after the scan, and if the reference acupressure pressure and the new acupressure pressure are the same, to perform a thermal massage according to the automatic mode, and if the reference acupressure pressure and the new acupressure pressure are different, after confirming the location where the new acupressure pressure matches the reference acupressure pressure while repeatedly moving a certain distance along the longitudinal direction based on the location where the heating moxibustion device has moved, to perform a thermal massage according to the automatic mode.
